# FASCICULE DE BREVET EUROPEEN

(11) **EP 1 659 492 B1**
(45) Date de publication et mention de la délivrance du brevet: **13.06.2007**
(21) Numéro de dépôt: 05292428.9
(22) Date de dépôt: 16.11.2005
(51) Int. Cl.: G06F 9/445, A61B 5/00

(54) **Appareil électronique autonome, notamment enregistreur médical ambulatoire, à microcontrôleur reprogrammable**
Autonome elektronische Vorrichtung, insbesondere ein ambulantes medizinisches Aufnahmegerät, mit einem reprogrammierbaren Mikrocontroller
Stand-alone electronic device, in particular an ambulatory medical recorder, with a reprogrammable microcontroller

(30) Priorité: 17.11.2004 FR 0412182
(43) Date de publication de la demande: 24.05.2006
(73) Titulaire: ELA MEDICAL, 92541 Montrouge (FR)
(72) Inventeur: Christophle-Boulard, Sylvain, 91700 Sainte Geneviève des Bois (FR); Faisandier, Yves, 75116 Paris (FR)
(74) Mandataire: Dupuis-Latour, Dominique

(56) Documents cités:
- FR-A- 2 693 007
- FR-A- 2 704 677
- US-A- 5 701 894

## Description

L'invention concerne les appareils électroniques autonomes comportant un microcontrôleur ou microprocesseur.

L'invention est particulièrement applicable aux appareils médicaux, notamment les enregistreurs ambulatoires d'électrocardiogrammes, qui permettent d'effectuer sur une longue période, en continu ou à la demande, le recueil et la mémorisation de signaux d'activité cardiaque à partir d'électrodes externes.

Cependant, comme on le comprendra, l'invention n'est pas limitée à ce type particulier d'appareil, et elle peut également concerner le recueil ou l'enregistrement d'autres données physiologiques telles que rythme respiratoire, tension artérielle, etc.

L'invention n'est d'ailleurs pas limitée aux appareils médicaux ; elle peut être appliquée à de très nombreux autres appareils électroniques, dès lors que ceux-ci sont pilotés par un microcontrôleur (ou un microprocesseur) reprogrammable et mettent en oeuvre une carte mémoire amovible pour le stockage d'un volume important de données : fichiers de données, d'images, de séquences sonores, etc.

Dans le cas des enregistreurs ambulatoires d'électrocardiogrammes, ces appareils sont aujourd'hui constitués d'un boîtier porté par le patient, par exemple à la ceinture ou dans une poche, et relié à un certain nombre d'électrodes ou autres capteurs externes. L'enregistreur est piloté par un microcontrôleur, et les données recueillies, brutes ou préalablement traitées, sont mémorisées sur un support de données amovible de grande capacité pour permettre un enregistrement intégral des données sur une très longue durée, typiquement d'un jour à plusieurs mois.

Aujourd'hui, les supports d'enregistrement magnétique (cassettes) ont été remplacés par des mémoires électroniques telles que des mémoires flash, qui sont à la fois réinscriptibles et non volatiles, et sont maintenant disponibles avec des capacités mémoire très importantes. Le FR-A-2 704 677 décrit un tel type de mémoire et expose une manière d'en assurer la reprogrammation (réinscription).

Un enregistreur à mémoire électronique comprend un boîtier pourvu sur le côté d'une ouverture par laquelle peut être insérée la carte mémoire amovible. Une fois l'enregistrement achevé, la carte contenant les données recueillies est extraite du boîtier et remise au praticien ou envoyée à un centre distant pour analyse des données qui y sont contenues, à des fins de diagnostic.

L'utilisation d'un microcontrôleur reprogrammable présente l'avantage de pouvoir faire évoluer l'appareil sans nécessiter le changement d'un composant ni même de l'appareil dans son ensemble. La reprogrammation du microcontrôleur permet de corriger d'éventuels défauts du programme et d'ajouter de nouvelles fonctionnalités aux appareils existants par de nouvelles versions du programme, en satisfaisant ainsi une clientèle très sensible au progrès.

La reprogrammation du microcontrôleur exige en général un branchement spécifique au microcontrôleur, défini par le constructeur du composant et comprenant le plus souvent une liaison série et plusieurs lignes de commandes associées. Le circuit imprimé supportant le microcontrôleur comporte un certain nombre de contacts destinées à la reprogrammation, qui doivent pouvoir être accessibles de l'extérieur sans démontage de l'appareil, par exemple via une ouverture formée dans le boîtier au droit des contacts de reprogrammation, l'ouverture devant pouvoir être occultée en permanence, en dehors des phases de reprogrammation au moyen d'un obturateur approprié.

Une autre technique, décrite dans le FR-A-2 693 007 (ELA Medical), consiste, pour éviter le recours à des contacts de reprogrammation, à incorporer le nouveau programme du microcontrôleur à une cartouche mémoire enfichable substituée à la cartouche de stockage des informations; le programme est ensuite transféré dans une mémoire interne de l'enregistreur.

L'enregistreur étant porté par le patient dans des circonstances diverses et pendant une longue durée, il peut être soumis à un environnement d'humidité, de poussières, etc. dont il est indispensable de protéger efficacement la mémoire amovible et le circuit se trouvant à l'intérieur du boîtier. L'environnement de l'appareil peut être également perturbateur par les champs électromagnétiques parasites et les risques de décharges électrostatiques. L'accès au connecteur de carte, ainsi qu'à toute autre ouverture du boîtier (notamment celle donnant accès aux contacts de reprogrammation), doit donc être protégé à l'encontre de ce type d'agressions de l'environnement.

Pour opérer la reprogrammation, l'ouverture donnant accès aux contacts de reprogrammation est découverte de manière à pouvoir accéder aux divers contacts. Sur ces contacts sont alors appliqués des signaux permettant de forcer le microcontrôleur en mode de reprogrammation, puis sont appliquées sur les diverses lignes de commande des combinaisons particulières de signaux selon une séquence prédéterminée permettant de piloter le microcontrôleur de la manière souhaitée.

Ces branchements spécifiques impliquent un nombre relativement élevé de contacts et de lignes de commande aboutissant au microcontrôleur, ce qui complique la conception de l'appareil, que l'on cherche à réaliser le plus petit possible, et avec le moins possible d'ouvertures extérieures, afin de répondre au mieux aux exigences générales des appareils portables.

L'un des buts de l'invention est de proposer une configuration particulière d'appareil électronique autonome permettant de s'affranchir de toute ouverture dédiée d'accès aux contacts de reprogrammation, et donc des problèmes de positionnement et d'occultation étanche d'une telle ouverture.

Un autre but de l'invention est de permettre une réduction du nombre de lignes de reprogrammation du microcontrôleur, et donc du nombre de contacts de reprogrammation, évitant ainsi de compliquer inutilement la conception du circuit électronique.

L'appareil de l'invention est du type général comportant : une circuiterie et un ensemble de composants électroniques incluant un microcontrôleur ou microprocesseur; un bloc connecteur définissant un volume intérieur, avec un orifice apte à recevoir une carte mémoire amovible, ce bloc connecteur comportant des bornes de contact à des surfaces de contact homologues de la carte mémoire ; un circuit imprimé portant les composants électroniques, la circuiterie et le bloc connecteur ; un boîtier logeant le circuit imprimé et comportant, au droit de l'orifice latéral du bloc connecteur, une ouverture d'insertion d'une carte mémoire amovible ; et une pluralité de contacts de reprogrammation placés sur le circuit imprimé, aptes à permettre l'application à la circuiterie, depuis l'extérieur de l'appareil, de signaux électriques de reprogrammation du microcontrôleur ou microprocesseur.

De façon caractéristique de l'invention, les contacts de reprogrammation sont disposés sur le circuit imprimé à distance desdites bornes de contact à la carte mémoire, dans l'espace occupé par le bloc connecteur sur ce circuit imprimé, et débouchent dans le volume intérieur du bloc connecteur, et l'appareil est essentiellement dépourvu d'ouverture dédiée d'accès aux contacts de reprogrammation, l'accès à ces contacts étant possible via ladite ouverture depuis ledit volume intérieur du bloc connecteur, ce volume étant libre de carte mémoire insérée dans l'appareil.

Avantageusement, l'appareil comprend en outre un circuit d'interfaçage entre, d'une part, les contacts de reprogrammation et, d'autre part, des lignes reliées à des broches prédéterminées du microcontrôleur ou microprocesseur, ces lignes étant en nombre supérieur à celui des contacts de reprogrammation. Il est ainsi possible de réduire le nombre de contacts de reprogrammation, par exemple à quatre contacts (avec un contact de masse, deux contacts d'entrée/sortie série, et un contact de commande de présence de reprogrammation) - voire à trois contacts (avec un contact de masse et deux contacts d'entrée/sortie série) au prix d'une complexification de l'électronique.

Il est également possible d'utiliser certaines lignes de la carte flash pour établir la ou les liaisons, comme les deux lignes série "SPI" de la carte SD qui, moyennant un système de multiplexage, peuvent supporter les transmission série.

Dans une configuration particulière, le bloc connecteur comprend une partie antérieure portant les bornes de contact à la carte mémoire et, entre cette partie antérieure et l'orifice latéral d'insertion de la carte mémoire, une partie comportant au moins une fenêtre formée entre le volume intérieur du bloc connecteur et le circuit imprimé, les contacts de reprogrammation étant disposés sur le circuit imprimé dans la région de ladite fenêtre.

Par ailleurs, les contacts de reprogrammation sont préférentiellement des plages de contact formées sur ledit circuit imprimé.

La présente invention a également pour objet, en tant que produit nouveau, un cordon de liaison, pour la connexion à une unité externe de l'appareil exposé ci-dessus, ce cordon comportant un câble multiconducteur pourvu à l'une de ses extrémités d'un insert dimensionné au format d'un carte mémoire amovible de manière à pouvoir être introduit dans l'appareil par ladite ouverture en lieu et place d'une carte mémoire, cet insert portant une pluralité d'organes de contact disposés en des positions respectives telles qu'ils viennent en contact avec les contacts de reprogrammation, lorsque l'insert est introduit dans l'appareil, chacun de ces organes de contact étant relié à un conducteur respectif du câble, soit directement soit par l'intermédiaire d'un circuit d'adaptation des caractéristiques des signaux échangés entre l'unité externe et le microcontrôleur ou microprocesseur.

L'appareil externe peut être notamment une unité de reprogrammation du microcontrôleur ou microprocesseur, ou une unité de diagnostic et de prise de contrôle de l'appareil.

On va maintenant décrire un exemple de mise en oeuvre du dispositif de l'invention, en référence aux dessins annexés où les mêmes références numériques désignent d'une figure à l'autre des éléments identiques.

La figure 1 est une vue générale d'un appareil électronique autonome auquel peut être appliquée la présente invention, avec sa carte mémoire amovible.

La figure 2 est une vue en plan du circuit imprimé de l'appareil de l'invention et des divers éléments qu'il supporte, avec une carte mémoire amovible et un cordon de liaison spécifique destiné à la reprogrammation.

La figure 3 est une vue partielle, en élévation et en coupe, du bloc connecteur présent sur le circuit de la figure 2, illustrant la manière dont il est possible d'insérer soit une carte mémoire amovible, soit un insert d'accès aux contacts de reprogrammation.

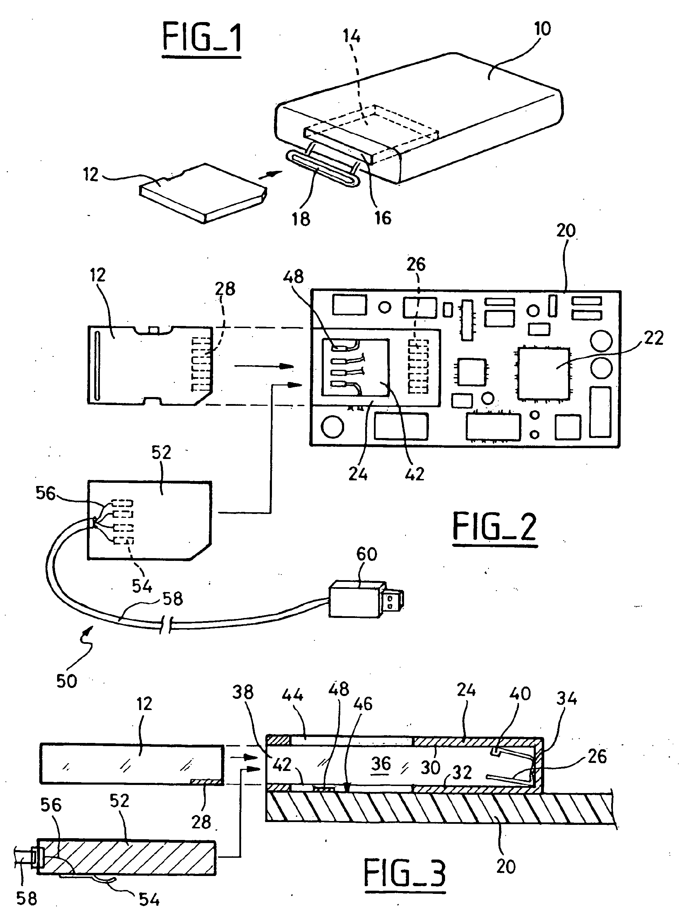

Sur la figure 1, la référence 10 désigne de façon générale un appareil électronique autonome, par exemple un enregistreur Holter de données électrocardiographiques tel que les modèles *Spiderview* ou *Spiderflash* commercialisés par ELA Medical, Montrouge, France.

Cet appareil permet l'enregistrement de données sur une carte mémoire amovible 12, qui est une mémoire flash de type standard, par exemple de type *SD Card.* Ce type de carte n'est bien entendu pas limitatif, et d'autres formats de cartes peuvent être utilisés : *CompactFlash, MultiMediaCard, XD Card,* etc.

La carte mémoire 12 est destinée à être insérée dans un logement 14 de l'appareil 10 par une ouverture 16, occultable de manière étanche au moyen d'un volet 18.

Comme illustré figure 2 et 3, l'appareil 10 contient un circuit imprimé 20 supportant et reliant un ensemble de composants électroniques avec notamment un microcontrôleur reprogrammable 22. Le circuit imprimé 20 supporte également un bloc connecteur 24 susceptible de recevoir la carte mémoire 12 et pourvu de bornes 26 de contact à des surfaces de contact homologues 28 de la carte mémoire 12.

Comme on peut le voir plus précisément sur la coupe de la figure 3, le bloc connecteur 24 est en forme de boîtier parallélépipédique, avec une face supérieure 30, une face inférieure 32 en contact avec le circuit imprimé 20, et des faces latérales 34, l'ensemble définissant un volume intérieur 36 susceptible de loger la carte 12. Le boîtier du bloc connecteur est ouvert sur le côté en 38 pour permettre l'insertion de la carte, et comporte à l'extrémité opposée à cette ouverture des moyens mécaniques 40 de verrouillage et de libération de la carte mémoire, ainsi que les bornes de contact 26.

Ce type de bloc connecteur 24, qui est un composant en lui-même bien connu et couramment disponible, comporte généralement une fenêtre 42 formée dans la face inférieure 32 et une fenêtre 44 formée dans la face supérieure 30.

Ces fenêtres 42 et 44 sont situées dans des parties non fonctionnelles du connecteur, à distance des bornes de contact 26, et laissent découverte une région 46 du circuit imprimé.

Selon l'invention, cette région découverte 46 est utilisée pour disposer des contacts de reprogrammation, auxquels il sera donc possible d'accéder via l'ouverture 16 normalement dédiée à l'insertion de la carte mémoire 12, après retrait de cette carte.

La prise de contact avec ces contacts de reprogrammation 48 peut notamment être réalisée au moyen d'un cordon de liaison 50 comportant un insert 52 dimensionné au format d'une carte mémoire (pour permettre son insertion dans le bloc connecteur 24 et son verrouillage mécanique à l'intérieur de celui-ci), mais comportant des lames de contact 54 sur sa face inférieure, disposées en des positions telles qu'elles viennent en contact avec les contacts de reprogrammation respectifs 48 lorsque l'insert 52 est introduit à fond dans le bloc connecteur 24. Chacune de ces lames 54 est reliée à un conducteur 56 d'un câble 58 du cordon 50, ce dernier se terminant par exemple, à son autre extrémité, par une fiche 60 permettant le branchement direct à l'unité de reprogrammation.

Dans certaines configurations, il peut être nécessaire d'interfacer les lignes du microcontrôleur avec la sortie du système de reprogrammation de manière plus complexe : c'est le cas par exemple d'une sortie USB de micro-ordinateur à relier avec une liaison série asynchrone ou synchrone, qui exige de placer un microcontrôleur dédié à la gestion USB. La ligne de raccordement sera alors équipée d'un circuit électronique chargé d'adapter les caractéristiques des signaux échangés avec l'unité de reprogrammation avec ceux du microcontrôleur, placé soit sur la prise de contact, soit à un autre niveau.

On peut de cette manière répondre à toutes les conditions exigées par la connexion spécifique à la reprogrammation, en utilisant l'ouverture dédiée à l'accès d'une carte mémoire pour implémenter la connectique correspondante.

On profite ainsi de l'étanchéité du volet d'occultation 18, et de la protection à l'encontre des champs électromagnétiques parasites et des risques de décharges électrostatiques, protection déjà assurée pour la carte mémoire amovible située à l'intérieur du boîtier.

L'optimisation de ce montage passe par une réduction du nombre de lignes de liaison au microcontrôleur, et donc du nombre de contacts de reprogrammation. En effet, étant donnée la petite taille du connecteur, il est intéressant de disposer d'un nombre de lignes aussi réduit que possible afin de ne pas compliquer inutilement le montage et rester dans les limites des fenêtres 42 et 44 du bloc connecteur 24.

Il est possible de réduire ainsi dans certains cas la liaison à quatre lignes, comprenant un ligne de masse, deux lignes série (aller/retour, pour une liaison asynchrone) et une ligne de présence pour forcer le microcontrôleur en mode de reprogrammation ; un circuit particulier permet simplement d'interfacer cette ligne de commande unique à la pluralité de lignes du microcontrôleur concernées par la reprogrammation.

On notera que ce nombre de quatre lignes n'est pas limitatif. Il est possible dans certains cas de les réduire à trois, en omettant la ligne spécifique de présence et en utilisant les deux lignes série en configuration particulière pour la transmission des commandes - cette réduction impliquant une complexification de l'électronique associée. Inversement, dans une configuration simplifiée il est possible de prévoir cinq lignes, avec deux lignes spécifiques de présence et/ou une ligne d'horloge (transmission synchrone), certaines pouvant être partagées avec les contacts de la carte mémoire elle-même.

Il est possible, tout en restant dans le cadre de la présente invention, d'utiliser les enseignements de l'invention pour d'autres applications que la reprogrammation. On peut notamment souhaiter piloter à la demande le microcontrôleur depuis l'extérieur, notamment à des fins de diagnostic ou de mise au point. Cette technique, dite d"'émulation *in situ*" du microcontrôleur, consiste à utiliser des lignes série, par exemple les lignes "JTAG", pour agir sur le microcontrôleur en permettant une lecture de tous les registres, un mode pas à pas, des points d'arrêt et la majorité des fonctions classiquement dédiées classiquement à un émulateur. En compliquant très peu ou même pas du tout l'interface avec les lignes décrites précédemment, il devient alors possible de commander de l'extérieur l'appareil, boîtier fermé, ce qui ouvre beaucoup de possibilités pour, d'une part, le tester en configuration finale et, d'autre part, lire des données que l'appareil aurait en mémoire mais que, par exemple, suite à un dysfonctionnement on ne pourrait plus lui extraire.

On comprendra à cet égard que l'utilisation du terme de "contacts de reprogrammation" dans la présente description n'implique aucune restriction de l'usage susceptible d'être fait de ces contacts : l'application de l'invention à la reprogrammation d'un microprocesseur ou microcontrôleur n'est aucunement restrictive, et l'invention peut aussi bien être utilisée dans le cadre d'autres applications, telles que l'"émulation *in situ*" évoquée plus haut.

## Revendications

1. Un appareil électronique autonome (10), comportant :
- une circuiterie et un ensemble de composants électroniques incluant un microcontrôleur ou microprocesseur (22),
- un bloc connecteur (24) définissant un volume intérieur (36), avec un orifice (38) apte à recevoir une carte mémoire (12) amovible, ce bloc connecteur comportant des bornes (26) de contact à des surfaces de contact homologues (28) de la carte mémoire (12),
- un circuit imprimé (20) portant les composants électroniques, la circuiterie et le bloc connecteur,
- un boîtier logeant le circuit imprimé et comportant, au droit de l'orifice latéral du bloc connecteur, une ouverture (16) d'insertion d'une carte mémoire amovible, et
- une pluralité de contacts de reprogrammation (48) placés sur le circuit imprimé, aptes à permettre l'application à la circuiterie, depuis l'extérieur de l'appareil, de signaux électriques de reprogrammation du microcontrôleur ou microprocesseur,
appareil **caractérisé en ce que** lesdits contacts de reprogrammation (48) sont disposés sur le circuit imprimé (20) à distance desdites bornes (26) de contact à la carte mémoire, dans l'espace (46) occupé par le bloc connecteur (24) sur ce circuit imprimé, et débouchent dans le volume intérieur (36) du bloc connecteur, et **en ce que** l'appareil est essentiellement dépourvu d'ouverture dédiée d'accès aux contacts de reprogrammation, l'accès à ces contacts étant possible via ladite ouverture depuis ledit volume intérieur du bloc connecteur, ce volume étant libre de carte mémoire insérée dans l'appareil.

2. L'appareil électronique autonome de la revendication 1, où l'appareil est un enregistreur médical ambulatoire.

3. L'appareil électronique autonome de la revendication 1, comprenant en outre un circuit d'interfaçage entre, d'une part, les contacts de reprogrammation et, d'autre part, des lignes reliées à des broches prédéterminées du microcontrôleur ou microprocesseur, ces lignes étant en nombre supérieur à celui des contacts de reprogrammation.

4. L'appareil électronique autonome de la revendication 3, comprenant au moins trois contacts de reprogrammation, avec un contact de masse et deux contacts d'entrée/sortie série.

5. L'appareil électronique autonome de la revendication 1, où le bloc connecteur (24) comprend une partie antérieure portant les bornes (26) de contact à la carte mémoire et, entre cette partie antérieure et l'orifice latéral (38) d'insertion de la carte mémoire, une partie comportant au moins une fenêtre (42, 46) formée entre le volume intérieur (36) du bloc connecteur et le circuit imprimé (20), les contacts de reprogrammation (48) étant disposés sur le circuit imprimé dans la région de ladite fenêtre.

6. L'appareil électronique autonome de la revendication 1, où lesdits contacts de reprogrammation (48) sont des plages de contact formées sur ledit circuit imprimé (20).

7. Un cordon de liaison (50), pour la connexion de l'appareil (10) selon l'une des revendications 1 à 6 à une unité externe, ce cordon comportant un câble multiconducteur (58) pourvu à l'une de ses extrémités d'un insert (52) dimensionné au format d'une carte mémoire amovible de manière à pouvoir être introduit dans l'appareil par ladite ouverture (16) en lieu et place d'une carte mémoire, cet insert (52) portant une pluralité d'organes de contact (54) disposés en des positions respectives telles qu'ils viennent en contact avec les contacts (48) de reprogrammation lorsque l'insert est introduit dans l'appareil, chacun de ces organes de contact étant relié à un conducteur respectif (56) du câble (58).

8. Le cordon de liaison de la revendication 7, où les organes de contact sont directement reliés aux conducteurs respectifs (56) du câble (58).

9. Le cordon de liaison de la revendication 7, où les organes de contact sont reliés aux conducteurs respectifs (56) du câble (58) par l'intermédiaire d'un circuit d'adaptation des caractéristiques des signaux échangés entre l'unité externe et le microcontrôleur ou microprocesseur.

10. Le cordon de liaison de la revendication 7, adapté à la connexion à une unité externe de reprogrammation du microcontrôleur ou microprocesseur.

11. Le cordon de liaison de la revendication 7, adapté à la connexion à une unité externe de diagnostic et de prise de contrôle de l'appareil.

## Claims

1. An autonomous electronic apparatus (10) comprising:
- circuitry and a set of electronic components including a microcontroller or microprocessor (22);
- a connector block (24) defining an inner volume (36) with an aperture (38) suitable for receiving a removable memory card (12), this connector block comprising terminals (26) for making contact with corresponding contact surfaces (28) of the memory card (12);
- a printed circuit (20) carrying the electronic components, the circuitry and the connector block;
- a casing housing the printed circuit and comprising, in line with the lateral aperture of the connector block, an opening (16) for inserting a removable memory card; and
- a plurality of reprogramming contacts (48) placed on the printed circuit and suitable for enabling electrical microcontroller or microprocessor reprogramming signals to be applied to the circuitry from outside the apparatus;
the apparatus being **characterized in that** said reprogramming contacts (48) are arranged on the printed circuit (20) at a distance from said terminals (26) for making contact with the memory card, in the space (46) occupied by the connector block (24) on the printed circuit, and open out into the inner volume (36) of the connector block, and **in that** the apparatus is essentially lacking any opening dedicated to the access to the reprogramming contacts, with access to said contacts being possible via said opening from said inner volume of the connector block, said volume being void of any memory card inserted in the apparatus.

2. The autonomous electronic apparatus of claim 1, wherein the apparatus is an ambulatory medical recorder.

3. The autonomous electronic apparatus of claim 1, further comprising an interface circuit between, on the one hand, the reprogramming contacts and, on the other hand, lines connected to predetermined pins of the microcontroller or microprocessor, the number of lines being greater than the number of reprogramming contacts.

4. The autonomous electronic apparatus of claim 3, comprising at least three reprogramming contacts, with a ground contact and two serial input/output contacts.

5. The autonomous electronic apparatus of claim 1, wherein the connector block (24) comprises a front portion carrying the terminals (26) for making contact with the memory card, and, between this front portion and the lateral memory card insertion aperture (38), a portion comprising at least one window (44, 46) formed between the inner volume (36) of the connector block and the printed circuit (20), the reprogramming contacts (48) being disposed on the printed circuit in the region of said window.

6. The autonomous electronic apparatus of claim 1, wherein said reprogramming contacts (48) are contact areas formed on said printed circuit (20).

7. A connection cord (50) for connecting the apparatus (10) according to any one of claims 1 to 6 to an external unit, this cord comprising a multi-conductor cable (58) provided at one of its ends with an insert (52) dimensioned to the format of a removable memory card so as to be capable of being inserted into the apparatus via said opening (16) instead of a memory card, this insert (52) carrying a plurality of contact members (54) disposed in respective positions so as to come into contact with the reprogramming contacts (48) when the insert is inserted into the apparatus, each of these contact members being connected to a respective conductor (56) of the cable (58).

8. The connection cord of claim 7, wherein the contact members are directly connected to the respective conductors (56) of the cable (58).

9. The connection cord of claim 7, wherein the contact members are connected to the respective conductors (56) of the cable (58) via a circuit for adapting the characteristics of the signals interchanged between the external unit and the microcontroller or microprocessor.

10. The connection cord of claim 7, adapted for the connection with an external microcontroller or microprocessor reprogramming unit.

11. The connection cord of claim 7, adapted for the connection with an external diagnosis unit for taking control of the apparatus.

## Patentansprüche

1. Autonome elektronische Vorrichtung (10), mit
- einer Schaltung und einer Gesamtheit von elektronischen Komponenten mit einem Mikrocontroller oder einem Mikroprozessor (22),
- einem Verbindungsblock (24), der ein Innenvolumen (36) definiert, mit einem Einlass (38), der geeignet ist, eine entfernbare Speicherkarte (12) aufzunehmen, wobei dieser Verbindungsblock Anschlüsse (26) für den Kontakt mit entsprechenden Kontaktflächen (28) der Speicherkarte (12) umfasst,
- einer gedruckten Schaltung (20), welche die elektronischen Komponenten, die Schaltung und den Verbindungsblock trägt,
- einem Gehäuse, welches die gedruckte Schaltung aufnimmt und auf der Höhe des Seiteneinlasses des Verbindungsblocks eine Öffnung (16) zum Einschub einer entfernbaren Speicherkarte umfasst, und
- einer Mehrzahl von Kontakten (48) zur Neuprogrammierung, die auf der gedruckten Schaltung angeordnet sind, die dazu geeignet sind, vom Äußeren der Vorrichtung aus auf die Schaltung die Anwendung von elektrischen Signalen zur Neuprogrammierung des Mikrocontrollers oder Mikroprozessor zu erlauben,
Vorrichtung, die **dadurch gekennzeichnet ist, dass** die Kontakte (48) zur Neuprogrammierung auf der gedruckten Schaltung (20) angeordnet sind, entfernt der Anschlüsse (26) für den Kontakt mit der Speicherkarte im durch den auf dieser gedruckten Schaltung vom Verbindungsblock (24) eingenommenen Raum (46), und in das Innenvolumen (36) des Verbindungsblocks münden, und **dadurch**, dass die Vorrichtung im Wesentlichen keine für den Zugang zu den Kontakten zur Neuprogrammierung eigens vorgesehene Öffnung aufweist, wobei der Zugang zu diesen Kontakten über die Öffnung ausgehend vom Innenvolumen des Verbindungsblocks möglich ist, wobei dieses Volumen frei von einer in die Vorrichtung eingeführten Speicherkarte ist.

2. Autonome elektronische Vorrichtung nach Anspruch 1, wobei die Vorrichtung ein tragbares medizinisches Aufnahmegerät ist.

3. Autonome elektronische Vorrichtung nach Anspruch 1, weiterhin mit einer Schnittstellenschaltung zwischen, einerseits, den Kontakten zur Neuprogrammierung und, anderseits, Leitungen, die mit vorbestimmten Kontakten des Mikrocontrollers oder Mikroprozessors verbunden sind, wobei diese Leitungen zahlreicher als die Kontakte zur Neuprogrammierung sind.

4. Autonome elektronische Vorrichtung nach Anspruch 3, mit mindestens drei Kontakten zur Neuprogrammierung, mit einem Erdungskontakt eine zwei seriellen Eingangs/Ausgangs-Kontakten.

5. Autonome elektronische Vorrichtung nach Anspruch 1, wobei der Verbindungsblock (24) einen vorderen Teil umfasst, welcher die Anschlüsse (26) für den Kontakt mit der Speicherkarte trägt, und zwischen diesem vorderen Teil und dem Seiteneinlass (38) zum Einführen der Speicherkarte einen Teil mit wenigstens einem Fenster (42, 46), das zwischen dem Innenvolumen (36) des Verbindungsblocks und der gedruckten Schaltung (20) ausgebildet ist, wobei die Kontakte (48) zur Neuprogrammierung auf der gedruckten Schaltung im Bereich des Fensters angeordnet sind.

6. Autonome elektronische Vorrichtung nach Anspruch 1, wobei die Kontakte (48) zur Neuprogrammierung Kontaktbereiche sind, die auf der gedruckten Schaltung (20) ausgebildet sind.

7. Verbindungskabel (50) zur Verbindung der Vorrichtung (10) nach einem der Ansprüche 1 bis 6 mit einer externen Einheit, wobei dieses Kabel ein Mehrleitungskabel (58) umfasst, das an einem seiner Enden mit einem Einschub (52) versehen ist, der Dimensionen gemäß dem Format einer entfernbaren Speicherkarte aufweist, um so anstelle einer Speicherkarte durch die Öffnung (16) in die Vorrichtung eingeführt werden zu können, wobei dieser Einschub (52) eine Mehrzahl von Kontaktelementen (54) trägt, die an entsprechenden Positionen angeordnet sind, so dass sie mit den Kontakten (48) zur Neuprogrammierung in Berührung kommen, wenn der Einschub in die Vorrichtung eingeführt wird, wobei jedes dieser Kontaktelemente mit einem jeweiligen Leiter (56) des Kabels (58) verbunden ist.

8. Verbindungskabel nach Anspruch 7, wobei die Kontaktelemente direkt mit den jeweiligen Leitern (56) des Kabels (58) verbunden sind.

9. Verbindungskabel nach Anspruch 7, wobei die Kontaktelemente mit den jeweiligen Leitern (56) des Kabels (58) über eine Schaltung zur Anpassung der Eigenschaften der zwischen der externen Einheit und dem Mikrocontroller oder Mikroprozessor ausgetauschten Signale verbunden sind.

10. Verbindungskabel nach Anspruch 7, geeignet zur Verbindung mit einer externen Einheit zur Neuprogrammierung des Mikrocontrollers oder Mikroprozessors.

11. Verbindungskabel nach Anspruch 7, geeignet zur Verbindung mit einer externen Einheit zur Diagnose und Übernahme der Steuerung der Vorrichtung.
